(19) Europäisches Patentamt
European Patent Office
Office européen des brevets

(11) **EP 3 502 847 A1**

(12) **DEMANDE DE BREVET EUROPEEN**

(43) Date de publication:
**26.06.2019 Bulletin 2019/26**

(51) Int Cl.:
*G06F 3/01* (2006.01)  *G06N 3/04* (2006.01)
*G06N 3/08* (2006.01)  *A61F 2/72* (2006.01)
*A61B 5/00* (2006.01)  *G06N 3/00* (2006.01)

(21) Numéro de dépôt: **18215081.3**

(22) Date de dépôt: **21.12.2018**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **22.12.2017 FR 1763070**

(71) Demandeur: **Commissariat à l'Énergie Atomique et aux Énergies Alternatives**
**75015 Paris cedex (FR)**

(72) Inventeur: **YELISYEYEV, Andriy**
**38100 Grenoble (FR)**

(74) Mandataire: **GIE Innovation Competence Group**
**310, avenue Berthelot**
**69372 Lyon Cedex 08 (FR)**

(54) **PROCÉDÉ D'OPTIMISATION DE L'APPRENTISSAGE D'UNE INTERFACE NEURONALE DIRECTE**

(57) L'invention est un procédé d'apprentissage par renforcement d'une interface neuronale directe, l'interface neuronale étant reliée à des électrodes préalablement disposées autour du cerveau d'un individu, chaque électrode étant configurée pour détecter un signal électrophysiologique et à adresser à l'interface neuronale un signal électronique représentatif du signal électrophysiologique détecté, l'interface étant configurée pour commander un actionneur en fonction de signaux électrophysiologiques détectés par chaque électrode, le procédé d'apprentissage comportant des étapes itératives, à chaque itération correspondant un instant d'itération (t), chaque itération comportant les étapes suivantes :
a) sélection d'une tache mentale ($T_i(t)$) à effectuer, choisie parmi une liste de tâches prédéterminée ($T_1(t)$ ..... $T_l(t)$);
b) instruction, à l'individu, de réaliser la tâche sélectionnée lors de l'étape a) ;
c) suite à la réalisation de la tâche, acquisition des signaux électroniques, issus des différentes électrodes;
d) décodage de signaux électroniques, de façon à identifier une composante des signaux électroniques spécifique de la tâche effectuée ;
e) réitération des étapes a) à e), en incrémentant l'instant d'itération ($t + 1$), jusqu'à l'atteinte d'un critère d'arrêt ;
le procédé étant caractérisé en ce que pour au moins une itération, l'étape a) est réalisée selon un apprentissage par renforcement.

**Fig. 1**

**Description**

**DOMAINE TECHNIQUE**

Le domaine technique de l'invention concerne le recours à des méthodes, de type apprentissage profond, pour optimiser un apprentissage d'une interface

**ART ANTERIEUR**

Le domaine des interfaces neuronales directes est en plein développement et apparaît comme une solution séduisante pour permettre à des personnes handicapées de commander des effecteurs par la pensée. Il s'agit d'enregistrer, généralement à l'aide d'électrodes corticales, des signaux électrophysiologiques. Ces derniers sont traités par des algorithmes permettant d'extraire un signal de commande, pour commander des actionneurs. Le signal de commande peut permettre le pilotage d'un exosquelette, d'un ordinateur ou d'un robot, pour fournir une assistance à l'utilisateur. Les algorithmes mis en oeuvre permettent une traduction d'une instruction donnée par l'utilisateur, cette instruction étant captée, par les électrodes, sous la forme de signaux dits électrophysiologiques, car représentatifs de l'activité électrique des neurones. Cette activité électrique est généralement mesurée au niveau du cortex, au moyen d'électrodes corticales disposées dans la boîte crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en oeuvre sont généralement basés sur un modèle prédictif. Le modèle prédictif utilise des données d'entrée, obtenues par un prétraitement des signaux électrophysiologiques enregistrés, pour établir un signal de commande, à destination d'un actionneur. Le signal de commande doit correspondre à une intention exprimée par l'utilisateur, c'est-à-dire l'individu dont on enregistre les signaux électrophysiologiques. L'intention exprimée par l'utilisateur se manifeste sous la forme de signaux électrophysiologiques, ces derniers étant enregistrés et transmis vers l'interface neuronale directe, formant des données d'entrée. Les données d'entrées sont traitées par le modèle prédictif pour générer le signal de commande.

Les données d'entrée sont généralement multidimensionnelles, et comprennent :

- une composante spatiale, représentative de l'origine spatiale du signal électrophysiologique ;
- une composante fréquentielle, représentative de l'intensité du signal électrophysiologique dans différentes bandes de fréquence ;
- une composante temporelle, représentative de l'évolution temporelle du signal électrophysiologique, chaque signal électrophysiologique étant acquis selon une fréquence d'échantillonnage élevé, typiquement quelques kHz, durant un intervalle temporel d'acquisition de quelques centaines de millisecondes à quelques secondes.

Le modèle prédictif est établi au cours d'une phase d'apprentissage, au cours de laquelle l'utilisateur effectue des tâches prédéfinies. L'objectif est alors, suite à chaque tâche, de déterminer des composantes des signaux électrophysiologiques enregistrés spécifiques de la tâche. Il peut notamment s'agir de déterminer des corrélations entre des composantes de signaux et la tâche réalisée. Les composantes des signaux peuvent être temporelles, spatiales, fréquentielles, ou leur combinaison.

L'établissement de modèles prédictifs a amplement été décrit. Par exemple, le brevet US9480583 décrit l'application d'une méthode de régression linéaire des moindres carrés partiels multivariée permettant d'établir un modèle prédictif. Une telle méthode est connue sous l'acronyme anglosaxon "NPLS" ou par le terme "N-way Partial Least Squares". L'application d'une telle méthode a également été décrite dans la publication Eliseyev A, Aksenova T (2013) "Recursive N-way Partial Least Squares for Brain Computer Interface" PIOS ONE july 2013, Volume 8 Issue 7 e69962. Une telle méthode est également décrite dans le document Yelisyeyev A "Brain-Computer Interface with cortical electrical activity recording" Human health and pathology. Université de Grenoble, 2011.

L'inventeur a constaté que l'ordre dans lequel les tâches sont proposées à l'utilisateur peut avoir une influence sur l'apprentissage. Cela fait l'objet de l'invention décrite ci-après.

**EXPOSE DE L'INVENTION**

Un premier objet de l'invention est un procédé d'apprentissage par renforcement d'une interface neuronale directe, l'interface neuronale directe étant reliée à des capteurs préalablement disposées autour du cerveau d'un individu, chaque capteur étant configurée pour détecter un signal électrophysiologique et à adresser à l'interface neuronale un signal électronique représentatif du signal électrophysiologique détecté, l'interface neuronale directe étant destinée à commander un actionneur en fonction de signaux électroniques transmis par chaque capteur, le procédé d'apprentissage comportant des étapes itératives, à chaque itération correspondant un instant d'itération, chaque itération comportant les étapes suivantes

a) sélection d'une tâche mentale à effectuer, choisie parmi une liste de tâches prédéterminée;
b) instruction, à l'individu, de réaliser la tâche sélectionnée lors de l'étape a) ;
c) suite à la réalisation de la tâche par l'individu, acquisition des signaux électroniques, issus des différents capteurs;
d) décodage de signaux électroniques, de façon à identifier au moins une composante des signaux électroniques spécifique de la tâche effectuée ;
e) réitération des étapes a) à e), en incrémentant l'instant d'itération, jusqu'à l'atteinte d'un critère d'arrêt ;

le procédé étant caractérisé en ce que chaque itération comporte, entre l'étape d) et l'étape e), une étape intercalaire, mise en oeuvre par un microprocesseur, comportant les sous-étapes suivantes :

i) un calcul d'une performance de la tâche sélectionnée lors de l'étape a), quantifiant le degré d'apprentissage de la tâche, à partir de la composante identifiée lors de l'étape d);
ii) une mise à jour d'une variable d'état, comportant, pour chaque tâche de la liste de tâche:

• la performance suite à l'itération dernièrement effectuée, dite itération courante ;
• une évolution de la performance suite à une dernière itération au cours de laquelle la tâche a été effectuée;

iii) une détermination d'un critère d'évaluation, dont la valeur dépend des performances de chaque tâche à l'issue de l'itération, et d'une chronologie des tâches effectuées au cours des itérations précédentes ;
iv) une détermination d'une fonction de valeur, pour évaluer une performance globale de l'apprentissage, la fonction de valeur prenant en compte la variable d'état mise à jour lors de la sous-étape ii) et le critère d'évaluation déterminé lors de la sous-étape iii) ;

de telle sorte que lors de l'étape a) de l'itération suivante, la tâche sélectionnée correspond à une tâche maximisant la fonction de valeur.

Avant la première itération, le procédé peut comporter une phase d'initialisation pour déterminer la première tâche à réaliser, initialiser la variable d'état et définir le calcul du critère d'évaluation.

La variable d'état peut comporter, pour chaque tâche:

- la performance à l'instant de l'itération ;
- une variation de la performance lors de la dernière itération durant laquelle la tâche a été mise en oeuvre ;

Le procédé peut comporter, à chaque itération :

- une détermination de la tâche dont l'indicateur d'inactivité est maximal.

Le procédé peut être tel que le critère d'évaluation est calculé, suite à chaque itération, comporte une combinaison arithmétique :

- d'un premier terme représentant un nombre d'itérations consécutives de la tâche sélectionnée lors de l'itération ;
- d'un deuxième terme représentant, le nombre d'itérations s'étant écoulées depuis une dernière réalisation de la tâche dont l'indicateur d'inactivité est maximal ;
- d'un troisième terme, représentant la performance de la tâche effectuée au cours de l'itération courante.

Le troisième terme peut avoir une valeur d'autant plus élevée que la tâche effectuée lors de l'itération courante présente une performance faible relativement aux performances des autres tâches. Le critère d'évaluation est alors désigné par le terme "critère d'évaluation minimal".

Le procédé peut comporter, à chaque itération, et pour chaque tâche de la liste de tâches, une détermination d'une variation de la performance lors de la dernière réalisation de la tâche. Le troisième terme peut alors avoir une valeur d'autant plus élevée que la tâche effectuée lors de l'itération courante présente une variation de performance élevée relativement aux variations des performances des autres tâches. Le critère d'évaluation est alors désigné par le terme critère d'évaluation moyen.

Le troisième terme peut également être une combinaison arithmétique :

- d'une première composante, ayant une valeur d'autant plus élevée que la tâche effectuée lors de l'itération courante présente une performance faible relativement aux performances des autres tâches ;
- d'une deuxième composante, a une valeur d'autant plus élevée que la tâche effectuée lors de l'itération courante présente une variation de performance élevée relativement aux variations des performances des autres tâches.

Le critère d'évaluation est alors désigné par le terme critère d'évaluation combiné.

La sous-étape iv) peut être réalisée en mettant en oeuvre un algorithme de type réseau de neurones profonds.

Un autre objet de l'invention est une interface neuronale directe, comportant :

- des capteurs, aptes à acquérir des signaux électrophysiologiques représentatifs d'une activité corticale et à transmettre des signaux électroniques représentatifs des signaux électrophysiologiques ;
- une unité de traitement, pour traiter les signaux électroniques transmis par les capteurs, de façon à mettre en oeuvre l'étape d) du premier objet de l'invention, de façon à estimer une corrélation entre les signaux électroniques et la tâche réalisée lors de l'étape b) du premier objet de l'invention ;
- un processeur, configuré pour mettre en oeuvre les sous-étapes i) à iv), du procédé selon le premier objet de l'invention, à partir de la corrélation établie lors de l'étape d).

Les capteurs peuvent notamment être des électrodes corticales ou des électrodes d'électroencéphalographie.

Un autre objet de l'invention est un support d'enregistrement, comportant des instructions pour la mise en oeuvre des sous-étapes i) à iv) d'un procédé objet du premier objet de l'invention. D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1 schématise une interface neuronale reliée à un utilisateur, et connectée à un processeur apte à mettre en oeuvre un procédé selon l'invention.

La figure 2A montre les principales étapes d'un procédé selon l'invention.

La figure 2B illustre un détail d'une étape décrite en lien avec la figure 2A.

La figure 3 représente des fonctions générées aléatoirement préalablement à des modélisations du procédé, chaque fonction étant représentative d'une évolution temporelle de la performance d'une tâche.

La figure 4A montre une évolution temporelle des performances de dix tâches au cours d'une première série d'essais. Les figures 4B et 4C montrent, pour chaque tâche, un nombre moyen de répétitions de la tâche au cours de l'apprentissage, ainsi qu'un critère d'inactivité moyen. La figure 4D est une comparaison de performances du procédé selon l'invention avec des procédés d'apprentissage au cours desquels chaque tâche est sélectionnée aléatoirement ou séquentiellement.

La figure 5A montre une évolution temporelle des performances de dix tâches au cours d'une deuxième série d'essais. Les figures 5B et 5C montrent, pour chaque tâche, un nombre moyen de répétitions de la tâche au cours de l'apprentissage, ainsi qu'un critère d'inactivité moyen. La figure 5D est une comparaison de performances du procédé selon l'invention avec des procédés d'apprentissage au cours desquels chaque tâche est sélectionnée aléatoirement ou séquentiellement.

La figure 6A montre une évolution temporelle des performances de dix tâches au cours d'une troisième série d'essais. Les figures 6B et 6C montrent, pour chaque tâche, un nombre moyen de répétitions de la tâche au cours de l'apprentissage, ainsi qu'un critère d'inactivité moyen. La figure 6D est une comparaison de performances du procédé selon l'invention avec des procédés d'apprentissage au cours desquels chaque tâche est sélectionnée aléatoirement ou séquentiellement.

## EXPOSE DE MODES DE REALISATION PARTICULIERS

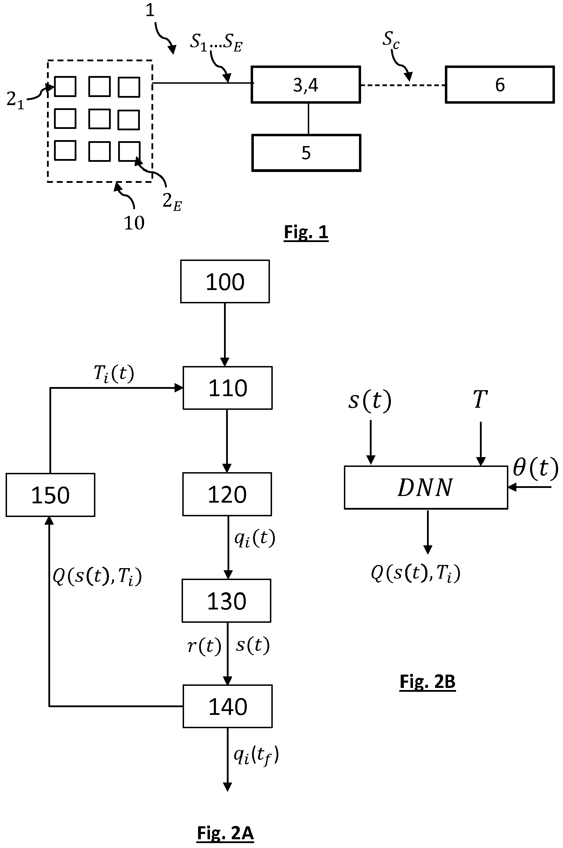

La figure 1 représente les principaux éléments d'une interface neuronale 1 selon l'invention. Il s'agit d'un dispositif comportant des capteurs $2_1...2_E$, permettant l'acquisition de signaux électrophysiologiques représentatifs d'une activité neuronale. Les capteurs $2_1...2_E$ sont par exemple des électrodes corticales, l'indice E désignant le nombre d'électrodes corticales. Les capteurs $2_1...2_E$ sont reliés à une unité de traitement 3, par exemple un microprocesseur, par une liaison filaire ou sans fil. Chaque capteur $2_1...2_E$ est configuré pour détecter un signal électrophysiologique émis par un utilisateur 10. A partir de chaque signal électrophysiologique détecté, chaque capteur $2_1...2_E$ transmet un signal électronique $S_1...S_E$ à l'unité de traitement. L'unité de traitement 3 est apte à mettre en oeuvre des algorithmes pour détecter des caractéristiques des signaux électroniques $S_1...S_E$ spécifiques à une tâche effectuée par le patient. L'unité de traitement 3 peut par exemple être un processeur relié à une mémoire mettant en oeuvre des instructions permettant d'effectuer des algorithmes de décodage tels que décrits dans les publications citées en lien avec l'art antérieur. Ces derniers permettent de décoder les signaux physiologiques détectés de façon à déterminer les caractéristiques des signaux électrophysiologiques corrélées des tâches mentales effectuées par l'utilisateur 10.

Par tâche mentale, désignée par la suite tâche, on entend une action imaginée par un utilisateur auquel l'interface neuronale directe est raccordée. Il peut s'agir par exemple d'imaginer bouger un membre d'une certaine façon, par exemple lever un bras ou baisser un bras. On peut également disposer un écran 5 devant l'utilisateur. L'écran comporte un curseur et l'utilisateur effectue des tâches mentales pour déplacer le curseur. Par exemple, l'utilisateur imagine déplacer une main vers la droite ou vers la gauche ou vers le haut ou vers le bas pour déplacer le curseur respectivement vers la droite, vers la gauche, vers le haut ou vers le bas.

Lors du fonctionnement opérationnel de l'interface neuronale directe 1, comme mentionné en relation avec l'art antérieur, l'utilisateur réalise successivement des tâches mentales. L'unité de traitement 3 reçoit les signaux électroniques $S_1...S_E$ transmis par les capteurs $2_1...2_E$, représentatifs des signaux électrophysiologiques produits par l'utilisateur et détectés par les capteurs. A partir des signaux électroniques détectés, lorsqu'une corrélation avec une tâche est détectée, le microprocesseur génère un signal de commande $S_c$ à l'attention d'un actionneur 6. Ainsi, l'interface neuronale directe décode les signaux électrophysiologiques produits par l'utilisateur 10 de façon à générer, à l'aide d'un modèle prédictif, des signaux de commande à un actionneur. La qualité du décodage est d'autant meilleure que l'algorithme de décodage a fait l'objet d'un apprentissage de qualité.

Au cours de l'apprentissage, l'utilisateur dispose d'une liste $T$ de tâches $T_i$ à effectuer. Comme décrit en lien avec l'art antérieur, au cours d'une phase d'apprentissage, on demande à l'utilisateur d'effectuer successivement des tâches $T_i$ choisies parmi la liste des tâches. L'objectif est de déterminer progressivement les caractéristiques électrophysiologiques les mieux corrélées avec les tâches. Ces caractéristiques permettent ensuite d'établir le modèle prédictif, mis en oeuvre lors du décodage, par lequel l'utilisateur 10 peut piloter l'actionneur 6 relié à l'unité de traitement 3.

Au cours de l'apprentissage, lors de chaque tâche, les signaux électrophysiologiques détectés dans un intervalle temporel suivant l'instruction donnée à l'utilisateur d'effectuer la tâche sont transmis, éventuellement après un prétraitement de type débruitage et/ou préamplification, à l'unité de traitement 3. Cette dernière procède au décodage, de façon à identifier les caractéristiques qui apparaissent les mieux corrélées avec tâche. L'apprentissage se répète, jusqu'à ce que l'on puisse disposer, pour chaque tâche, d'une certaine répétabilité dans les caractéristiques spécifiques de la tâche.

L'ordre selon lequel les tâches sont demandées à l'utilisateur, peut être séquentiel : on demande alors à l'utilisateur d'effectuer une tâche après une autre, selon un certain ordre, et on répète les tâches dans le même ordre durant l'apprentissage. La séquence de tâche peut également être aléatoire, chaque tâche à effectuer étant sélectionnée par un tirage aléatoire dans la liste des tâches.

L'inventeur propose une alternative différente, basée sur l'affectation, à chaque tâche, d'un indicateur de performance, quantifiant la performance d'apprentissage. L'indicateur de performance quantifie par exemple un niveau de corrélation entre au moins une caractéristique des signaux enregistrés et la tâche. La séquence de tâches suivant une itération dépend alors des performances établies pour chaque tâche, au cours des itérations précédentes.

L'invention met en oeuvre un processeur 4, par exemple identique à l'unité de traitement 3 préalablement décrite, de façon à établir automatiquement une séquence de tâches, en se basant sur un critère d'évaluation, décrit ci-après. Le processeur peut en particulier mettre en oeuvre un algorithme d'apprentissage par renforcement. L'apprentissage par renforcement est une technique permettant d'établir une séquence de tâches successives de façon à maximiser un indicateur de performance globale, noté $G$. Lors de la réalisation de chaque tâche, le procédé mis en oeuvre par le microprocesseur 4 génère un critère d'évaluation $r$, usuellement désigné par le terme anglosaxon "reward".

Le processeur peut être relié à l'écran 5, ou une autre interface homme-machine, de façon à indiquer à l'utilisateur une tâche à effectuer.

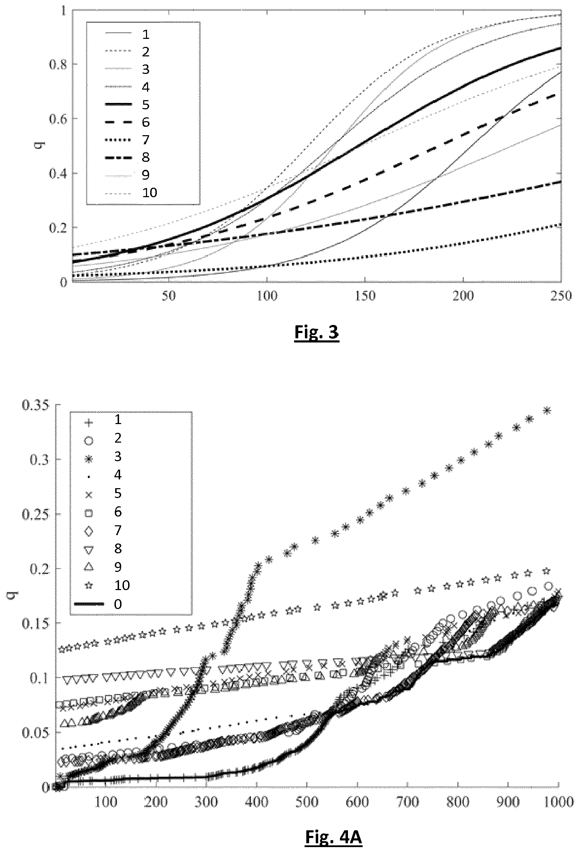

Le procédé mis en oeuvre est itératif, et se déroule selon des incréments temporels. A chaque itération correspond un instant $t$, dit instant d'itération. Par la suite, la lettre $t$ désigne le rang de chaque itération. Lors de chaque itération, l'algorithme détermine une tâche $T_i$ parmi la liste de tâches $T$. L'indice $i$ est un entier positif, avec $1 < i < I$, $I$ représentant le nombre de tâches de la liste de tâche $T$. A chaque itération $t$, est associée une variable d'état $s(t)$. Lors de chaque itération, à chaque tâche $T_i$ est associée une performance $q_i(t)$. Une performance $q_i(t)$ est dans cet exemple un scalaire. La performance $q_i(t)$ est représentative du niveau d'apprentissage d'une tâche $T_i$ à l'instant $t$. Elle peut par exemple dépendre d'un niveau de corrélation entre une caractéristique des signaux détectés et la tâche. Lors de l'apprentissage, la performance d'une tâche $q_i(t)$ suit de préférence une fonction croissante, de telles fonctions étant décrites par la suite en lien avec la figure 3.

L'invention se distingue de l'art antérieur par le fait que les tâches demandées à l'utilisateur suite à une itération $t$ dépendent de la performance $q_i(t)$ établie pour chaque tâche $T_i$, préalablement à l'itération, à travers la variable d'état $s(t)$.

La variable d'état déterminée lors de chaque itération comporte des informations relatives aux performances de chaque tâche ainsi qu'à une chronologie des tâches réalisées depuis le début de l'apprentissage. Dans l'exemple, la variable d'état $s(t)$ est une variable multidimensionnelle. Elle comporte les informations suivantes :

- la performance $q_i(t)$ de chaque tâche $T_i$ à l'instant $t$;
- pour chaque tâche $T_i$, une variation de la performance $\Delta q_i(t)$ au cours de la dernière itération au cours de de laquelle la tâche $T_i$ a été réalisée ;
- pour chaque tâche $T_i$, un nombre d'itérations $m_i(t)$ depuis lequel la tâche n'a pas été réalisée. Ce nombre d'itérations correpond à un indicateur d'activité de la tâche courante, c'est-à-dire de la tâche réalisée au cours de l'opération courante.

La dimension de la variable d'état est 3$l$.

Par exemple, si on ne considère que 2 tâches : $T = \{T_1, T_2\}$, si $s(t) = [(0.5 , 0.3) ; (0.1 ; 0.05) ; (0, 3)]$, cela représente le fait que :

- les performances respectives de $T_1$ et $T_2$ sont 0.5 et 0.3 ;
- lors de la dernière réalisation des tâches $T_1$ et $T_2$, leur performance respective a été améliorée de 0.1 et 0.05 ;
- lors de la dernière itération, la tâche $T_1$ a été mise en oeuvre, la tâche $T_2$ n'ayant pas été mise en oeuvre depuis 3 itérations.

L'objectif de l'algorithme est d'établir une séquence de tâche $\pi$ maximisant un indicateur de performance global. De façon usuelle dans les algorithmes de type apprentissage par renforcement, l'indicateur de performance globale $G(t)$, à un instant $t$, peut être exprimé par :

$$G(t) = r(t + 1) + \gamma r(t + 1) + \gamma^2 r(t + 2) \ldots = \sum_{k=0}^{K} \gamma^k r(t + k + 1) \ (1)$$

où :

- k désigne un incrément temporel ;
- $\gamma$ est un coefficient de pondération avec $0 < \gamma < 1$ ; ce coefficient permet de moduler l'importance de l'évaluation résultant des instants immédiatement postérieurs à l'instant $t$, par rapport à des instants plus lointains ;
- $r(t)$ est le critère d'évaluation de l'apprentissage établi lors de chaque itération. Le critère d'évaluation quantifie une progression de l'apprentissage lors de chaque itération. Ce critère est établi a priori, en fonction d'un objectif assigné à la séquence d'apprentissage. Par exemple, il peut s'agir de privilégier une progression de la tâche dont la performance est minimale. Il peut également s'agir de privilégier une performance moyenne des tâches. Le critère d'évaluation $r(t)$ est établi lors de chaque itération $t$, et peut être considéré comme une réponse du système subissant l'apprentissage à l'exécution d'une tâche à l'instant $t$. Par la suite, le critère d'évaluation est parfois désigné par le terme "évaluation".

L'indicateur de performance global correspond à une somme pondérée des évaluations de chaque tâche future.

La séquence optimale est apprise de façon implicite en prenant en compte une fonction de valeur $Q(s(t), T)$, selon un algorithme de $Q$-learning ($Q$-apprentissage), tel que décrit dans a publication Sutton R.S,"Reinforcement learning : an introduction", 2001. La fonction de valeur représente la somme des évaluations futures espérées en exécutant une tâche $T_i(t)$, depuis un état $s(t)$, en suivant la séquence de tâches $\pi$. Lors de chaque itération $t$, la fonction de valeur est mise à jour, suite à l'exécution d'une tâche $T_i(t)$, par l'équation de mise à jour :

$$Q\big(s(t), T_i(t)\big) \leftarrow Q\big(s(t), T_i(t)\big) + \alpha \left[ r(t + 1) + \gamma \max_{T_j \in T}(Q\big(s(t + 1), T_j\big)) - Q\big(s(t), T_i(t)\big) \right] (2)$$

où :

- $\leftarrow$ désigne l'opérateur de mise à jour ;
- $\alpha$ est un coefficient d'apprentissage, tel que $0 < \alpha < 1$ ;
- $\gamma$ est le coefficient de pondération décrit dans l'expression (1) ;
- $r(t + 1)$ correspond à l'évaluation suite à l'exécution de la tâche $T_i(t)$.
- $Q(s(t), T_i(t))$ est la fonction de valeur au cours de l'itération t ;

$$\max_{T_j \in T}(Q(s(t+1), T_j)) - Q(s(t), T_i(t))$$

- correspond à l'amélioration de la fonction de valeur lorsqu'on effectue la tâche $T_i(t)$. La fonction de valeur $Q(s(t), T_i(t))$ est obtenue, à chaque itération, par réseaux de neurones profonds, comme décrit ci-dessous. Lors de la première itération ($t = 1$), la variable d'état peut être initialisée en considérant une performance initiale de chaque tâche fixée à une valeur arbitraire, par exemple égale à 0. Au cours d'une période d'initialisation, correspondant à une itération, ou à plusieurs itérations, les tâches à effectuer peuvent être sélectionnées arbitrairement ou aléatoirement parmi la liste de tâches $T$.

L'estimation de la fonction de valeur $Q(s(t), T_i(t))$ peut être effectuée, au cours de chaque itération, en mettant en oeuvre un réseau de neurones profonds *DNN,* par exemple à deux couches, comme décrit dans la publication Sutton précédemment citée. A l'issue de chaque itération *t,* on incrémente l'instant d'itération et on sélectionne la tâche l'itération suivante permettant de maximiser la fonction de valeur, de telle sorte que $$T_i(t) = \operatorname*{argmax}_{T_j \in T}\left(Q(s(t), T_j)\right),$$ chaque fonction de valeur $Q(s(t), T_j)$, pour les différentes tâches $T_j$, étant estimée par *DNN.*

La sélection d'une tâche peut également inclure une part aléatoire. Ainsi, selon une variante, la sélection de la tâche $T_i(t)$ est réalisée :

- selon une probabilité $(1-\varepsilon)$, en mettant en oeuvre l'équation (3) ;
- aléatoirement, selon une probabilité $\varepsilon$.

La figure 2A représente les principales étapes d'un procédé selon l'invention.

Etape 100 : initialisation des paramètres, détermination de la liste des tâches et prise en compte d'un critère d'évaluation r. Durant cette étape, la variable d'état est initialisée. Le critère d'évaluation r, qui sera mis en oeuvre au cours de chaque itération, est choisi en fonction de l'objectif que l'on s'est attribué.

Les étapes 110 à 150 sont ensuite réalisées de façon itérative, à chaque instant d'itération t. Une itération en cours de réalisation est dénommée itération courante.

Etape 110 : en fonction d'une sélection d'une tâche initiale, ou résultant d'une itération précédente, on donne l'instruction à l'utilisateur 10 de réaliser la tâche sélectionnée $T_i(t)$ parmi la liste des tâches T.

Etape 120 : l'utilisateur réalise la tâche $T_i(t)$, dite tâche courante. Les signaux électrophysiologiques résultant de la réalisation sont détectés par les électrodes durant une période temporelle, généralement de quelques secondes, suivant l'instruction. L'unité de traitement détermine une performance de la tâche $q_i(t)$ à partir des signaux électrophysiologiques détectés.

Etape 130 : calcul de l'évaluation $r(t)$. Le calcul de l'évaluation dépend de l'objectif que l'on s'est assigné lors de l'étape 100. L'expression analytique de l'évaluation $r(t)$ dépend de cet objectif.

Selon un premier exemple, l'objectif peut être de privilégier les gains de performances de la tâche dont la performance est minimale. Le critère d'évaluation $r(t)$ répondant à cet objectif, désigné par la notation $r_{min}(t)$, peut être obtenu par une combinaison arithmétique, par exemple un produit, de trois termes $\rho_1(t)$, $\rho_2(t)$ et $\rho_{min}(t)$.

- Le premier terme $\rho_1(t)$ a pour objectif de pénaliser une réalisation successive d'une même tâche. Pour cela, on affecte à la tâche courante $T_i$ un nombre $n_i(t)$ correspondant aux nombres d'itérations, antérieures à l'instant courant *t,* au cours desquelles la tâche courante a été effectuée consécutivement. Par exemple, $\rho_1(t) = e^{-\kappa_1 n_i(t)}$ (4), $\kappa_1$ étant un réel positif ou nul. Lorsque $\kappa_1 = 0$, on ne pénalise pas qu'une même tache courante soit réitérée.
- Le deuxième terme $\rho_2(t)$ a pour objectif de pénaliser la non réalisation de tâches depuis un grand nombre d'itérations. Pour cela, on affecte à chaque tâche un indicateur d'inactivité correspondant au nombre d'itérations $m_i(t)$ s'étant écoulées depuis une dernière réalisation de ladite tâche. On détermine ensuite la valeur maximale de l'indicateur d'inactivité $$m(t) = \max_{T_i}(m_i(t)). \rho_2$$ dépend de $m(t)$. Par exemple, $\rho_2(t) = e^{-\kappa_2 m(t)}$ (5), $\kappa_2$ étant un réel positif ou nul. Lorsque $\kappa_2 = 0$, on ne pénalise pas la non réalisation d'une tâche au cours d'un grand nombre d'itérations.
- Le troisième terme $\rho_{min}(t)$ prend en compte la performance $q_i(t)$ de la tâche courante, relativement aux performances des autres tâches à l'instant courant. $\rho_{min}(t) \in [0,1]$. $\rho_{min}(t) = 1$ lorsque la performance de la tache courante est inférieure aux performances des autres tâches, ce qui va tendre à favoriser la sélection de cette tâche lors de l'itération suivante. $\rho_{min}(t) = 0$ lorsque la performance de la tache courante est supérieure aux performances des autres tâches, ce qui va tendre à favoriser la sélection d'une autre tâche lors de l'itération suivante.

Le critère d'évaluation $r_{min}(t)$ peut ainsi prendre la forme

$$r_{min}(t) = \rho_1(t)\,\rho_2(t)\,\rho_{min}(t) \;\text{(6)},$$

correspondant à un produit des trois termes définis ci-avant.

Selon un deuxième exemple, l'objectif peut être de privilégier un gain de performance de tâches, c'est-à-dire favoriser la réalisation de tâches considérées comme en progrès, pour lesquelles la variation de performance $\Delta q_i(t)$ est élevée. Le critère d'évaluation $r$ répondant à cet objectif, désigné par la notation $r_{avr}(t)$, peut être obtenu par une combinaison arithmétique, par exemple un produit, de trois termes $\rho_1(t)$, $\rho_2(t)$ et $\rho_{avr}(t)$. $\rho_1(t)$, $\rho_2(t)$ sont les premier et deuxième termes explicités ci-avant, en lien avec les expressions (4) et (5). $\rho_{avr}(t) \in [0,1]$. $\rho_{avr}(t) = 1$ lorsque la variation $\Delta q_i(t)$ de la performance de la tache courante est supérieure à la variation des performances des autres tâches, ce qui va tendre à favoriser la sélection de la tâche courante lors de l'itération suivante. $\rho_{avr}(t) = 0$ lorsque la variation de la performance de la tache courante est inférieure aux variations des performances des autres tâches, ce qui va tendre à favoriser la sélection d'une autre tâche lors de l'itération suivante.

L'évaluation $r_{avr}(t)$ peut ainsi prendre la forme

$$r_{avr}(t) = \rho_1(t)\,\rho_2(t)\,\rho_{avr}(t) \quad \text{(7)},$$

correspondant à un produit des trois termes définis ci-avant.

Selon un troisième exemple, les deux critères précédents, définis en lien avec les expressions (6) et (7) sont combinés, de façon à obtenir un critère d'évaluation combiné $r_{cmp}(t)$, avec

$$r_{cmp}(t) = \rho_1(t)\,\rho_2(t)\left(\frac{\rho_{avr}(t)+\rho_{min}(t)}{2}\right)\;\text{(8)}$$

Des simulations d'applications respectives de $r_{min}(t)$, $r_{avr}(t)$ et $r_{cmp}(t)$ sont présentées en lien avec les figures 4A à 4D, 5A à 5D et 6A à 6D.

L'étape 130 comporte également une mise à jour du vecteur d'état $s(t)$ en fonction de la performance de la tâche courante $q_i(t)$ déterminée lors de l'étape 120.

Etape 140 : mise à jour de la fonction de valeur $Q(s(t), T_i(t))$, définie en lien avec l'équation (2), de façon à sélectionner la tâche à effectuer lors de l'itération suivante

La fonction de valeur est une fonction non linéaire et sa mise à jour peut être réalisée en mettant en oeuvre un réseau de neurones profond *DNN* (Deep Neural Network), comme décrit en lien avec la figure 2B. Les données d'entrée du *DNN* sont le vecteur d'état $s(t)$, mis à jour lors de l'étape 130, et la liste des tâches $T$. L'objectif est de déterminer la tâche maximisant la fonction de valeur, comme décrit en lien avec l'équation (3), cette tâche étant ensuite affectée à l'itération suivante.

Le réseau de neurones peut comprendre deux couches cachées successives de $4I$ neurones. Il est paramétré par un vecteur $\theta(t)$, de dimension $K$, dont chaque terme correspond à facteur de pondération d'une connexion du réseau de neurones. Le vecteur $\theta(t)$ comporte des termes $w_k$, avec $1 \le k \le K$. Le vecteur $\theta(t)$ est un paramètre caché de la fonction de valeur $Q$, cette dernière pouvant être exprimée en fonction de $\theta(t)$.

La mise à jour du vecteur $\theta(t)$, lors de l'étape 140, peut être réalisée selon les expressions :

$$\theta(t+1) = \theta(t) + \alpha\left[r(t+1) + \gamma \max_{T_i \in T} Q\big(s(t+1), T_i, \theta(t)\big) - Q\big(s(t), T_i(t), \theta(t)\big)\right] e(t)\;\text{(9)}$$

où $0 < \lambda < 1$ et $e(t)$ est un vecteur de dimension $K$ tel que

$$e(t) = \lambda\,\gamma\,e(t-1) + \nabla_\theta\big(Q\big(s(t), T_i(t), \theta(t)\big)\;\text{(10)}.$$

$\nabla_\theta$ désigne un gradient, selon le vecteur $\theta$, de la fonction de valeur. Le terme $\nabla_\theta Q(s(t), T_i(t), \theta(t))$ peut être estimé en mettant en oeuvre un algorithme de rétropropagation.

Le vecteur $\theta$ est initialisé avant la première itération.

<u>Etape 150</u> : on estime la tâche $T_i(t)$ à réaliser lors de l'itération suivante en mettant en oeuvre l'équation (3) en

$$T_i(t) = \underset{T_j \in T}{\text{argmax}} \left( Q\big(s(t), T_j\big) \right).$$

maximisant la fonction de valeur : Les fonctions de valeur $Q(s(t), T_j)$ sont calculées pour chaque tâche $T_j$ de la liste de tâche.

Les itérations se poursuivent en incrémentant l'instant d'itération jusqu'à l'atteinte d'un critère d'arrêt d'itération. Ce dernier peut être l'atteinte d'un niveau de performance d'une tâche, ou l'atteinte d'un nombre d'itérations préderminé. A l'issue de la période d'apprentissage, $t = t_f$. On dispose des performances $q_i(t_f)$ de chaque tâche $T_i$ à l'instant final $t = t_f$

Le procédé décrit en lien avec les figures 2A et 2B a été modélisé, en considérant respectivement les critères d'évaluation $r_{min}(t)$,$r_{avr}(t)$ et $r_{cmp}(t)$. Pour réaliser les simulations, on a établi des fonctions paramétriques $q_i$ de l'évolution des performances de dix tâches $T_i$. Les modèles paramétriques sont de la forme :

$$q_i(t) = \frac{1}{1 + e^{-A_i t + B_i}} \,(11)$$

Les paramètres $A_i$ et $B_i$ sont des réels positifs déterminés aléatoirement pour chaque tâche $T_i$ en considérant :

- pour $A_i$, une distribution selon une loi normale $\mathcal{N}$ (0.025 ; 0.01)

- pour $B_i$, une distribution selon une loi normale $\mathcal{N}$ (2.5 ; 1).

$q_i(t) \in [0,1]$·

La figure 3 montre un exemple de 10 fonctions ainsi générées. L'axe des ordonnées représente la fonction $q_i(t)$, l'axe des abscisses représentant les incréments temporels, compris entre $t=0$ et $t=250$. Les fonctions $q_i(t)$ sont représentatives de fonctions d'apprentissage, car elles sont croissantes et commencent à des niveaux initiaux différents. Certaines fonctions croissent plus rapidement que d'autres, car certaines tâches s'apprennent plus rapidement que d'autres.

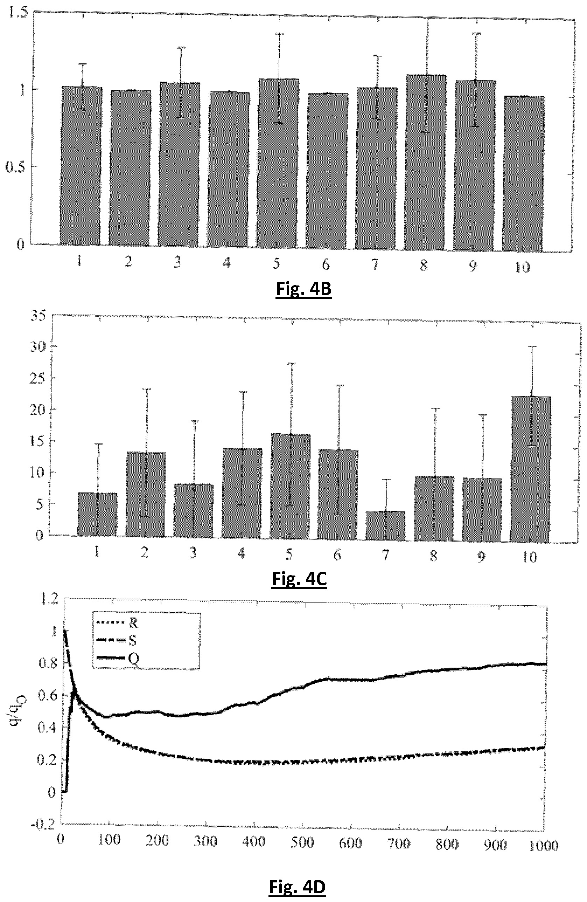

Les figures 4A, 4B, 4C et 4D représentent les résultats obtenus suite à une première série de modélisations. Au cours de ces modélisations, on a effectué l'algorithme tel que décrit en lien avec les figures 2A et 2B, en utilisant l'évaluation $r_{min}(t)$ précédemment décrite. Il s'agit d'un apprentissage $Q$ (Q learning). Pour la mise en oeuvre de l'algorithme, les paramètres préalablement évoqués sont les suivants : $\varepsilon = 0.3$, $\lambda = 0.9$ , $\gamma = 0.5$, $\alpha = 0.1$ ; $\kappa_1 = 0.5$ ; $\kappa_2 = 0.03$.

Les fonctions de performance étant connues, car il s'agit des fonctions paramétriques commentées en lien avec la figure 3, une stratégie optimale a été déterminée.

La figure 4A montre, pour chaque tâche, l'évolution de la performance $q_i(t)$ de chaque tâche $T_i$ au cours de 1000 itérations, l'axe des abscisses représentant les itérations. La ligne en trait plein, repérée par le chiffre 0, correspond à la performance minimale $\underset{T_i \in T}{\min} q_i(t)$ à chaque itération. Ce graphe montre l'évolution de l'apprentissage au cours des itérations successives.

La figure 4B représente, pour chaque tâche, le nombre moyen de répétitions d'une même tâche au cours de deux itérations successives. On rappelle que ce nombre moyen est conditionné par le premier terme de pénalisation $\rho_1(t)$ de l'évaluation $r_{min}(t)$. Ce nombre est voisin de 1 pour chaque tâche.

La figure 4C représente, pour chaque tâche, le nombre moyen d'inactivité $m_i(t)$ de la tâche $T_i$, c'est-à-dire le nombre moyen d'itérations entre deux réalisations successives de la tâche réalisé au cours de l'itération $t$. On rappelle que ce nombre moyen est conditionné par le premier terme de pénalisation $\rho_2(t)$ de l'évaluation $r_{min}(t)$. Ce nombre est fluctuant pour chaque tâche.

On a également testé un apprentissage en choisissant des tâches aléatoirement (apprentissage R) ou de façon séquentielle (apprentissage S). L'apprentissage séquentiel correspond à une réalisation des tâches dans un ordre prédéfini, l'une après l'autre. Pour chaque apprentissage, y compris l'apprentissage $Q$ selon l'invention, on a déterminé, à chaque itération, un ratio, noté $\frac{q}{q_0}$, entre la performance de l'algorithme, à chaque itération, déterminée par $\underset{T_i \in T}{\min} q_i(t),$ et la performance de l'algorithme optimal, cette dernière correspondant à la valeur optimale à atteindre. La figure 4D représente l'évolution du ratio, pour chaque type d'apprentissage, en fonction du nombre d'itérations. On constate que dès que le nombre d'itérations est supérieur à 200, l'apprentissage $Q$ permet d'obtenir une performance plus élevée. Les performances des apprentissages aléatoire et séquentiel sont du même ordre de grandeur, les courbes représentées sur la figure 4D étant confondues.

Ainsi, en mettant en oeuvre un apprentissage *Q,* objet de l'invention, l'obtention d'un niveau de performance acceptable, pour chaque tâche, requiert moins de temps qu'avec une stratégie d'apprentissage classique, de type séquentielle ou aléatoire. A l'issue d'une même durée d'apprentissage, la performance d'apprentissage obtenue avec l'invention est plus élevée. Cela un permet, suite à l'apprentissage, lors d'une utilisation opérationnelle de l'interface neuronale, un meilleur contrôle de l'actionneur 6 par l'interface neuronale.

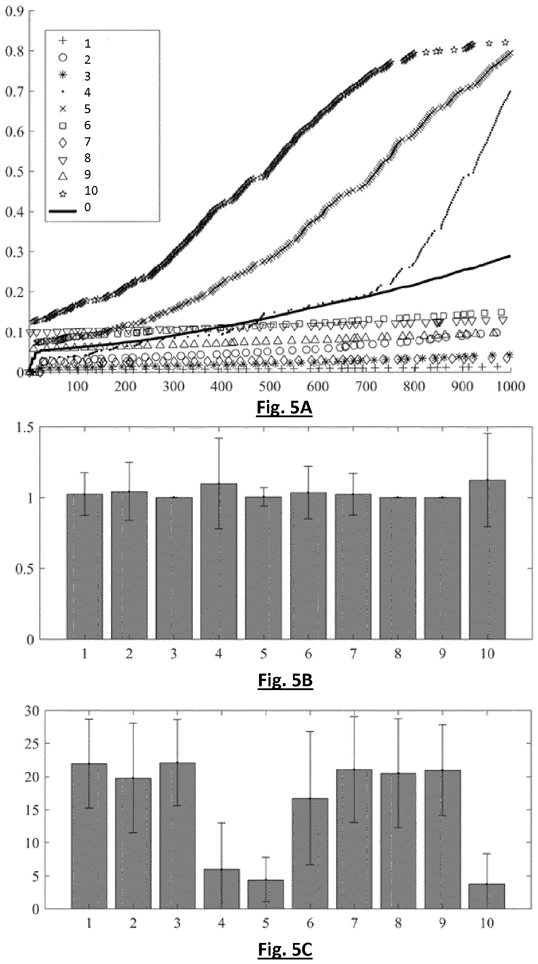

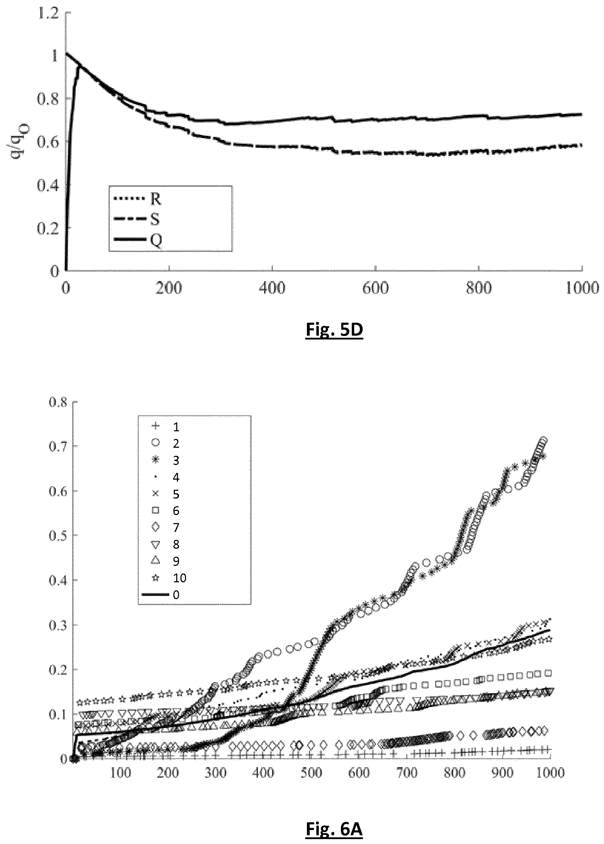

Les figures 5A, 5B, 5C et 5D représentent les résultats obtenus suite à une deuxième série de modélisations. Au cours de ces modélisations, on a effectué l'algorithme tel que décrit en lien avec les figures 2A et 2B, en utilisant l'évaluation $r_{avr}(t)$ précédemment décrite.Les figures 5A, 5B, 5C et 5D sont analogues aux figures 4A, 4B, 4C et 4D. Sur la figure 5A, la performance de l'algorithme, représentée par un trait plein (indice 0 sur l'échelle), correspond à une moyenne $\underset{T_i \in T}{\text{mean}} \, q_i(t)$ des performances de chaque tâche.

Lors de chaque itération, les performances respectives de l'algorithme *Q* (représenté sur les figures 2A et 2B), R et S ont été normalisées par une performance optimale de l'algorithme. La figure 5D montre l'évolution temporelle des performances normalisées $\frac{q}{q_O}$. De même qu'en lien avec la figure 4D, on observe que le procédé objet de l'invention permet d'atteindre une meilleure performance d'apprentissage.

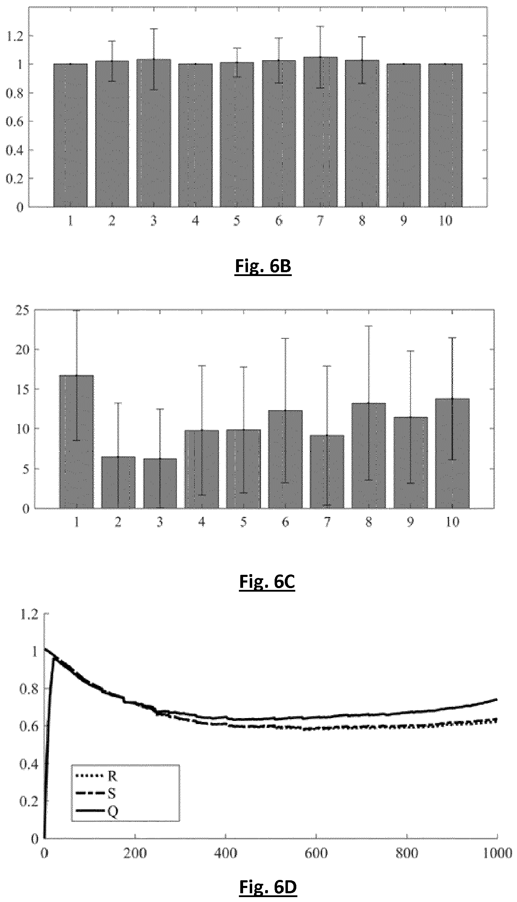

Les figures 6A, 6B, 6C et 6D représentent les résultats obtenus suite à une troisième série de modélisations. Au cours de ces modélisations, on a effectué l'algorithme tel que décrit en lien avec les figures 2A et 2B, en utilisant l'évaluation $r_{cmp}(t)$ précédemment décrite.Les figures 6A, 6B, 6C et 7D sont analogues aux figures 4A, 4B, 4C et 4D et 5A, 5B, 5C et 5D. Sur la figure 6A, la performance de l'algorithme, représentée par un trait plein (indice 0 sur l'échelle), correspond à une combinaison $\frac{1}{2}\left(\underset{T_i \in T}{\min} \, q_i(t)\right) + \frac{1}{2}\left(\underset{T_i \in T}{\text{mean}} \, q_i(t)\right)$ des performances de chaque tâche.

Lors de chaque itération, les performances respectives de l'algorithme *Q* (représenté sur les figures 2A et 2B), R et S ont été normalisées par une performance optimale $\frac{q}{q_O}$ de l'algorithme.

La figure 6D montre l'évolution temporelle des performances normalisées. De même qu'en lien avec les figures 4D et 5D, on observe que le procédé objet de l'invention permet d'atteindre une meilleure performance d'apprentissage.

L'invention pourra être mise en oeuvre afin d'améliorer le processus d'apprentissage d'une interface neuronale directe, de telle sorte qu'on obtienne plus rapidement un modèle prédictif de qualité, permettant une meilleure commande de l'actionneur 6, par l'utilisateur 10, lors du fonctionnement opérationnel de l'interface.

## Revendications

1. Procédé d'apprentissage par renforcement d'une interface neuronale directe (1), l'interface neuronale directe étant reliée à des capteurs ($2_1...2_E$) préalablement disposés autour du cerveau d'un individu, chaque capteur étant configurée pour détecter un signal électrophysiologique et à adresser à l'interface neuronale un signal électronique ($S_1...S_E$) représentatif du signal électrophysiologique détecté, l'interface étant configurée pour commander un actionneur (6) en fonction de signaux électroniques transmis par chaque capteur, le procédé d'apprentissage comportant des étapes itératives, à chaque itération correspondant un instant d'itération (*t*), chaque itération comportant les étapes suivantes :

   a) sélection d'une tache mentale ($T_i(t)$) à effectuer, choisie parmi une liste de tâches prédéterminée ($T_1(t).....T_I(t)$);
   b) instruction, à l'individu, de réaliser la tâche sélectionnée lors de l'étape a) ;
   c) suite à la réalisation de la tâche par l'individu, acquisition des signaux électroniques, issus des différents capteurs;
   d) décodage de signaux électroniques, de façon à identifier une composante des signaux électroniques spécifique de la tâche effectuée ;
   e) réitération des étapes a) à e), en incrémentant l'instant d'itération (*t* + 1), jusqu'à l'atteinte d'un critère d'arrêt ;

   le procédé étant **caractérisé en ce que** chaque itération comporte, entre l'étape d) et l'étape e), une étape intercalaire, mise en oeuvre par un microprocesseur, comportant les sous étapes suivantes :

i) un calcul d'une performance ($q_i(t)$) de la tâche sélectionnée lors de l'étape a), quantifiant le degré d'apprentissage de la tâche, à partir de la composante identifiée lors de l'étape d) ;

ii) une mise à jour d'une variable d'état ($s(t)$), comportant, pour chaque tâche de la liste de tâche:

• la performance suite à l'itération dernièrement effectuée, dite itération courante ;
• une évolution de la performance suite à une dernière itération au cours de laquelle la tâche a été effectuée;

iii) une détermination d'un critère d'évaluation ($r(t + 1)$), dont la valeur dépend des performances ($q_i(t)$) de chaque tâche à l'issue de l'itération, et d'une chronologie des tâches effectuées au cours des itérations précédentes ;

iv) une détermination d'une fonction de valeur ($Q(s(t)$, $T_i(t)$), pour évaluer une performance globale de l'apprentissage, la fonction de valeur prenant en compte la variable d'état mise à jour lors de la sous-étape ii) et le critère d'évaluation ($r(t + 1)$) déterminé lors de la sous-étape iii) ;

de telle sorte que lors de l'étape a) de l'itération suivante, la tâche sélectionnée correspond à une tâche maximisant la fonction de valeur.

2. Procédé selon la revendication 1, dans lequel la variable d'état $s(t)$ comporte, pour chaque tâche:

- la performance ($q_i(t)$) à l'instant de l'itération ;
- une variation de la performance lors de la dernière itération durant laquelle la tâche a été mise en oeuvre ;
- le nombre d'itérations s'étant écoulé depuis une dernière réalisation de la tâche, ce dernier correspondant à un critère d'inactivité ($m_i(t)$).

3. Procédé selon la revendication 2, comportant, à chaque itération :

- une détermination de la tâche dont l'indicateur d'inactivité ($m_i(t)$) est maximal ;

le procédé étant tel que le critère d'évaluation ($r(t)$) est calculé, suite à chaque itération, comporte une combinaison arithmétique :

- d'un premier terme représentant un nombre d'itérations consécutives de la tâche sélectionnée lors de l'itération ;
- d'un deuxième terme représentant, le nombre d'itérations s'étant écoulées depuis une dernière réalisation de la tâche dont l'indicateur d'inactivité est maximal ;
- d'un troisième terme, représentant la performance de la tâche effectuée au cours de l'itération courante.

4. Procédé selon la revendication 3, dans lequel le troisième terme a une valeur d'autant plus élevée que la tâche effectuée lors de l'itération courante présente une performance faible relativement aux performances des autres tâches, le critère d'évaluation ($r(t)$) étant alors un critère d'évaluation minimal ($r_{min}(t)$).

5. Procédé selon la revendication 3, comportant, à chaque itération, et pour chaque tâche de la liste de tâches, une détermination d'une variation de la performance lors de la dernière réalisation de la tâche, et dans lequel le troisième terme a une valeur d'autant plus élevée que la tâche effectuée lors de l'itération courante présente une variation de performance élevée relativement aux variations des performances des autres tâches, le critère d'évaluation ($r(t)$) étant alors un critère d'évaluation moyen ($r_{avr}(t)$).

6. Procédé selon la revendication 3 comportant, à chaque itération, et pour chaque tâche de la liste de tâches, une détermination d'une variation de la performance lors de la dernière réalisation de la tâche ;
et dans lequel le troisième terme est une combinaison arithmétique :

- d'une première composante, ayant une valeur d'autant plus élevée que la tâche effectuée lors de l'itération courante présente une performance faible relativement aux performances des autres tâches ;
- d'une deuxième composante, a une d'autant plus élevée que la tâche effectuée lors de l'itération courante présente une variation de performance élevée relativement aux variations des performances des autres tâches ;

le critère d'évaluation ($r(t)$) étant un critère d'évaluation ($r_{cmp}(t)$) dit combiné.

7. Procédé selon l'une quelconque des revendications précédentes, dans lequel la sous-étape iv) est réalisée en mettant en oeuvre un algorithme de type réseau de neurones profonds (*DNN*).

8. Interface neuronale directe (1), comportant :

- des capteurs ($2_1...2_E$), aptes à acquérir des signaux électrophysiologiques représentatifs d'une activité corticale et à transmettre des signaux électroniques ($S_1...S_E$) représentatifs des signaux électrophysiologiques ;
- une unité de traitement (3), pour traiter lesdits signaux électroniques ($S_1...S_E$) transmis par les capteurs, de façon à mettre en oeuvre l'étape d) d'un procédé selon l'une quelconque des revendications précédentes, de façon à estimer une corrélation entre les signaux électroniques et la tâche réalisée lors de l'étape b) d'un procédé selon l'une quelconque des revendications précédentes.
- un processeur (4), configuré pour mettre en oeuvre les sous-étapes i) à iv), du procédé objet de l'une quelconque des revendications précédentes, à partir de la corrélation établie lors de l'étape d).

9. Support d'enregistrement, comportant des instructions pour la mise en oeuvre des sous-étapes i) à iv) d'un procédé objet de l'une quelconque des revendications 1 à 7.

$S_1...S_E$  $S_c$

$2_1$  3,4  6

5

$2_E$

10

**Fig. 1**

100

$T_i(t)$  110

150  120

$Q(s(t),T_i)$  $q_i(t)$

130

$r(t)$ $s(t)$

140

$q_i(t_f)$

**Fig. 2A**

$s(t)$  $T$

$DNN$  $\theta(t)$

$Q(s(t),T_i)$

**Fig. 2B**

**Fig. 3**

**Fig. 4A**

**Fig. 4B**

**Fig. 4C**

**Fig. 4D**

**Fig. 5A**

**Fig. 5B**

**Fig. 5C**

**Fig. 5D**

**Fig. 6A**

**Fig. 6B**

**Fig. 6C**

**Fig. 6D**

Europäisches
Patentamt

European
Patent Office

Office européen
des brevets

# RAPPORT DE RECHERCHE EUROPEENNE

Numéro de la demande

EP 18 21 5081

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | JOAN FRUITET ET AL: "Automatic motor task selection via a bandit algorithm for a brain-controlled button", JOURNAL OF NEURAL ENGINEERING, INSTITUTE OF PHYSICS PUBLISHING, BRISTOL, GB, vol. 10, no. 1, 21 janvier 2013 (2013-01-21), page 16012, XP020238286, ISSN: 1741-2552, DOI: 10.1088/1741-2560/10/1/016012 * abrégé * * chapitres "1. Introduction" - "2. Material and methods"; pages 1-5; figures 1-4; tableau 1 * ----- | 1-9 | INV.<br>G06F3/01<br>G06N3/04<br>G06N3/08<br>A61F2/72<br>A61B5/00<br>G06N3/00 |
| A | Anonymous: "Q-learning - Wikipedia", , 16 décembre 2017 (2017-12-16), XP055512611, Extrait de l'Internet: URL:https://en.wikipedia.org/w/index.php?title=Q-learning&oldid=815760432 [extrait le 2018-10-05] * le document en entier * ----- | 1-9 | DOMAINES TECHNIQUES RECHERCHES (IPC)<br><br>G06F<br>G06N<br>A61F<br>A61B |

Le présent rapport a été établi pour toutes les revendications

| Lieu de la recherche | Date d'achèvement de la recherche | Examinateur |
|---|---|---|
| La Haye | 13 février 2019 | Hasnas, Sergiu |

EPO FORM 1503 03.82 (P04C02)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• US 9480583 B

**Littérature non-brevet citée dans la description**

• **ELISEYEV A ; AKSENOVA T.** Recursive N-way Partial Least Squares for Brain Computer Interface. *PlOS ONE,* Juillet 2013, vol. 8 (7), e69962

• Brain-Computer Interface with cortical electrical activity recording. **YELISYEYEV A.** Human health and pathology. Université de Grenoble, 2011
• **SUTTON R.S.** *Reinforcement learning : an introduction,* 2001